# EUROPEAN PATENT APPLICATION

(11) **EP 4 775 676 A1**
(43) Date of publication of application: **15.07.2026**
(21) Application number: 24862860.4
(22) Date of filing: 05.09.2024
(51) Int. Cl.: C12N 15/55, A23L 31/15, C12N 1/15, C12N 1/19, C12N 1/21, C12N 5/10, C12N 9/16, C12N 15/63, C12P 1/00, C12P 19/32

(54) **PHOSPHODIESTERASE AND USE THEREOF**

(30) Priority: 06.09.2023 JP 2023144802; 15.11.2023 JP 2023194225
(71) Applicant: Amano Enzyme Inc., Nagoya-shi Aichi 460-8630 (JP)
(72) Inventor: SAKAI, Kiyota, Kakamigahara-shi, Gifu 509-0109 (JP); TAKABA, Nanase, Kakamigahara-shi, Gifu 509-0109 (JP)
(74) Representative: J A Kemp LLP
(86) International application number: PCT/JP2024/031834
(87) International publication number: WO 2025/053209

(57) **Abstract**

The present invention provides a phosphodiesterase derived from the genus Leptographium, containing the amino acid sequence represented by SEQ ID NO:1, or an amino acid sequence having 60% or more identity with the amino acid sequence, and having high GMP production capacity even in the presence of a high-concentration nucleic acid.

## Description

### [Technical Field]

The present invention relates to a novel phosphodiesterase and use thereof. More particularly, the present invention relates to a novel phosphodiesterase derived from the genus Leptographium and having superior 5'-guanosine monophosphate (GMP) production capacity even under high substrate concentration conditions, and use thereof.

### [Background Art]

In recent years, the share of plant-based alternative foods and reduced-salt foods has been growing in the food market. To improve the light taste of these products, nucleic acid-based seasonings such as yeast extract and the like are widely used as umami components. It is known that the umami of yeast extract is primarily due to 5'-guanosine monophosphate (GMP) contained in the extract.

For the purpose of enhancing umami, a method of producing GMP by reacting nucleic acid in yeast with phosphodiesterase is generally used as a means to increase the amount of GMP contained in yeast extract. As such phosphodiesterase, those derived from the genus Penicillium are widely used (Patent Literature 1).

Many enzymes, including phosphodiesterase, are known to exhibit a phenomenon ("substrate inhibition") in which, when substrate is present beyond a certain limit value, excessive binding of the substrate to the active site of the enzyme occurs to prevent the appropriate reaction, resulting in reduced enzymatic activity. The same is true for phosphodiesterase derived from the genus Penicillium, and the presence of nucleic acid in excess as a substrate tends to decrease the activity. Therefore, when yeast with a high nucleic acid concentration is used as a raw material in the production of yeast extract, it has been difficult to efficiently produce yeast extract containing a large amount of GMP.

### [Citation List]

### [Patent Literature]

[Patent Literature 1]
JP 2022-119910 A

### [Summary of Invention]

### [Technical Problem]

In view of this background, the present invention aims to provide a novel phosphodiesterase capable of highly producing GMP even under high substrate concentration conditions, and a method for producing a GMP-containing composition such as yeast extract and the like efficiently using the enzyme.

### [Solution to Problem]

The present inventors have conducted intensive studies in an attempt to achieve the above-mentioned goals and found that phosphodiesterase derived from the genus Leptographium has extremely superior GMP production capacity even under high substrate concentration conditions, compared to phosphodiesterase derived from the genus Penicillium, and conducted further studies based on the finding, which resulted in the completion of the present invention.

That is, the present invention provides the following.
[1] A phosphodiesterase derived from the genus Leptographium, comprising the amino acid sequence represented by SEQ ID NO:1, or an amino acid sequence having 60% or more identity with the amino acid sequence, and having high GMP production capacity even in the presence of a high-concentration nucleic acid.
[2] The phosphodiesterase of [1], wherein the genus Leptographium is Leptographium procerum.
[3] The phosphodiesterase of [1] or [2], wherein
   the amount of GMP produced is 20 mg/ml or more when an enzyme reaction is performed under the following conditions:
   <conditions>
   ·ribonucleic acid concentration: 40 mg/ml
   ·reaction temperature: 70°C
   ·reaction time: 180 min
   ·pH of the reaction mixture: 5.5
   ·amount of phosphodiesterase added per 1 mg of substrate RNA :60.8 U/mg.
[4] An enzyme agent for degrading a composition with high nucleic acid content, comprising the phosphodiesterase of any of [1] to [3].
[5] The enzyme agent of [4], wherein the concentration of the nucleic acid in the composition with high nucleic acid content is 35 mg/mL or more.
[6] An enzyme agent for producing a composition comprising 5'-guanosine monophosphate, comprising the phosphodiesterase of any of [1] to [3].
[7] The enzyme agent of [6], wherein the composition comprising 5'-guanosine monophosphate is yeast extract.
[8] A method for producing a composition comprising 5'-guanosine monophosphate, comprising contacting the phosphodiesterase of any of [1] to [3] or the enzyme agent of any of [4] to [7] with a composition comprising a nucleic acid.
[9] The production method of [8], wherein the ratio of the amount of the nucleic acid in the composition to the activity of phosphodiesterase is 0.001 U to 1000 U per 1 mg of the nucleic acid.
[10] The production method of [9], wherein the concentration of the nucleic acid in the composition is 35 mg/mL or more.
[11] The production method of any of [8] to [10], wherein the composition comprising the 5'-guanosine monophosphate is yeast extract.
[12] A GMP-containing composition produced by reacting the phosphodiesterase of any of [1] to [3] or the enzyme agent of any of [4] to [7] with a nucleic acid.
[13] A nucleic acid represented by SEQ ID NO:3 or 4, or a nucleic acid having 60% or more sequence identity with the nucleic acid.
[14] A vector comprising the nucleic acid of [13].
[15] A cell comprising the vector of [14].

### [Advantageous Effects of Invention]

According to the present invention, GMP can be produced efficiently from a nucleic acid even under high substrate concentration conditions. According to the present invention, moreover, compositions containing GMP, including yeast extract, can be produced under reaction conditions that could not be adopted when using conventional enzymes.

### [Description of Embodiments]

The present invention is described in detail in the following.

### 1. Phosphodiesterase

The present invention provides a phosphodiesterase derived from the genus Leptographium, comprising the amino acid sequence represented by SEQ ID NO:1, or an amino acid sequence having 60% or more identity with the amino acid sequence, and having high GMP production capacity even in the presence of a high-concentration nucleic acid (hereinafter sometimes referred to as "phosphodiesterase of the present invention").

Phosphodiesterase is an enzyme that hydrolyzes one of phosphodiester bonds. The phosphodiesterase of the present invention is a phosphodiesterase derived from the genus Leptographium and characterized by its resistance to substrate inhibition even in the presence of high concentrations of substrate nucleic acids, and by high phosphodiesterase activity thereof (particularly GMP-producing activity in the present invention).

Many species of the genus Leptographium, from which the phosphodiesterase of the present invention is derived, can affect wood and plant health and are attracting attention in the fields of forestry and plant protection. Examples of the genus Leptographium include, but are not limited to, Leptographium procerum, Leptographium terebrantis, Leptographium wingfieldii, Leptographium abietinum, Leptographium serpens, Leptographium lundbergii, Leptographium wageneri, Leptographium longiclavatum, Leptographium sinense, Leptographium murrayi, and the like. In one preferred embodiment of the present invention, the genus Leptographium may be Leptographium procerum. More preferably, it may be Leptographium procerum NBRC110178 strain. This strain is available from the National Institute of Technology and Evaluation (NITE) Biotechnology Center (NBRC).

The phosphodiesterase of the present invention is characterized by high resistance to substrate inhibition. The ratio of substrate (i.e., nucleic acid) to phosphodiesterase at which the phosphodiesterase of the present invention can exhibit its properties is not particularly limited as long as the desired effect is achieved. It is generally 1000U or less, preferably, 900U or less, 800U or less, 700U or less, 600U or less, 500U or less, 400U or less, 300U or less, 200U or less, 100U or less, 90U or less, 80U or less, 70U or less, 60U or less, 50U or less, 45U or less, 40U or less, 35U or less, 30U or less, or 25U or less, more preferably 20U or less, 15U or less, 13.5U or less, 10U or less, or 5U or less, per 1 mg of nucleic acid. The lower limit is not particularly limited as long as the desired effect is achieved, and may be generally 0.01U or more, preferably 0.1U or more, 1U or more, or 5U or more, per 1 mg of nucleic acid. In one embodiment, the ratio of substrate (nucleic acid):phosphodiesterase is, but not limited to, generally 0.01 to 1000U, preferably 0.1 to 500U, 1 to 300U, 5 to 100U, 10 to 90U, 10 to 60U, 10 to 40U, or 10 to 20U, per 1 mg of nucleic acid.

In the present specification, as phosphodiesterase activity (particularly ribonuclease activity), the amount of enzyme that liberates 1 µmol of phosphoric acid per minute when phosphodiesterase (particularly ribonuclease) is allowed to act on 3'-AMP as a substrate is defined as 1U.

The production capacity of 5'-guanosine monophosphate (sometimes referred to as "GMP" in the present specification) of the phosphodiesterase of the present invention under high substrate concentrations is higher than that of existing phosphodiesterases. In one embodiment, the phosphodiesterase of the present invention can also be defined using GMP production capacity under high substrate concentrations, as shown below:
<conditions 1>
   ·ribonucleic acid concentration: 40 mg/ml
   ·reaction temperature: 70°C
   ·reaction time: 180 min
   ·pH of the reaction mixture: 5.5
   ·amount of phosphodiesterase added per 1 mg of substrate RNA: 60.8U
   ·GMP produced: 15 mg/mL or more (preferably 16 mg/mL or more, more preferably 17 mg/mL or more)
<conditions 2>
   ·ribonucleic acid concentration: 60 mg/ml
   ·reaction temperature: 70°C
   ·reaction time: 180 min
   ·pH of the reaction mixture: 5.5
   ·amount of phosphodiesterase added per 1 mg of substrate RNA: 40.5 U
   ·GMP produced: 20 mg/mL or more (preferably 21 mg/mL or more, more preferably 22 mg/mL or more)

In another embodiment, the phosphodiesterase of the present invention can be defined as follows.
<conditions 3>
·ribonucleic acid concentration: 120 mg/ml
·reaction temperature: 70°C
·reaction time: 180 min
·pH of the reaction mixture: 5.5
·amount of phosphodiesterase added per 1 mg of substrate RNA: 20.3U
·GMP produced: 19 mg/mL or more (preferably 20 mg/mL or more, more preferably 21 mg/mL or more)

In another embodiment, the phosphodiesterase of the present invention can be defined as follows.
<conditions 4>
·ribonucleic acid concentration: 180 mg/ml
·reaction temperature: 70°C
·reaction time: 180 min
·pH of the reaction mixture: 5.5
·amount of phosphodiesterase added per 1 mg of substrate RNA: 13.5U
·GMP produced: 13 mg/mL or more (preferably 14 mg/mL or more, more preferably 15 mg/mL or more)

In another embodiment, the phosphodiesterase of the present invention has at least 2.6-fold higher GMP production capacity under the following conditions than control phosphodiesterase.
<conditions 5>
·ribonucleic acid concentration: 240 mg/ml
·reaction temperature: 70°C
·reaction time: 180 min
·pH of the reaction mixture: 5.5
·amount of phosphodiesterase added per 1 mg of substrate RNA: 10.1U
·GMP produced: 8 mg/mL or more (preferably 9 mg/mL or more, more preferably 10 mg/mL or more)

Furthermore, the phosphodiesterase of the present invention contains or consists of the amino acid sequence represented by SEQ ID NO:1, or an amino acid sequence having 60% or more, preferably 70% or more, 80% or more, or 90% or more (more preferably 91% or more, 92% or more, 93% or more, 94% or more, 95% or more, 96% or more, 97% or more, 98% or more, or 99% or more) identity with the amino acid sequence. The phosphodiesterase containing or consisting of the amino acid sequence having 60% or more, preferably 70% or more, 80% or more, or 90% or more (more preferably 91% or more, 92% or more, 93% or more, 94% or more, 95% or more, 96% or more, 97% or more, 98% or more, or 99% or more) identity with the amino acid sequence represented by SEQ ID NO:1 may be a phosphodiesterase having GMP production capacity equal to or greater than that of the phosphodiesterase consisting of the amino acid sequence represented by SEQ ID NO: 1. The identity (%) of amino acid sequences can be determined by a method known per se, for example, by calculation using sequence comparison software such as Basic Local Alignment Search Tool (BLAST).

As shown in the following Examples, the active center of the phosphodiesterase of the present invention is the seven amino acids (DSYRATA (SEQ ID NO:5), D and R are the binding sites) located at the 45-position to the 51-position of the amino acid sequence represented by SEQ ID NO:1 (corresponding to the 65-position to the 71-position in SEQ ID NO:2). Therefore, the phosphodiesterase of the present invention may be the amino acid sequence represented by SEQ ID NO:1 in which amino acids at the 45-position to the 51-position are not substituted and amino acids are substituted at other positions.

The phosphodiesterase derived from the genus Leptographium of the present invention can be prepared by a method known per se. The phosphodiesterase derived from the genus Leptographium can be prepared by separating from a culture medium of the genus Leptographium. The phosphodiesterase of the present invention can also be prepared by using genetic recombination technique or genome editing technique to introduce a nucleic acid encoding the phosphodiesterase of the present invention into a microorganism for protein expression and culturing the microorganism. One example of the preparation method includes a method of recovering the target phosphodiesterase from the culture medium or bacterial cells of the genus Leptographium. When phosphodiesterase is secreted from the bacterial cells, the bacterial cells can be removed from the culture medium by filtration or centrifugation as necessary, and the phosphodiesterase can be separated and/or purified from the culture medium from which the bacterial cells have been removed. When phosphodiesterase is not secreted from the bacterial cells, the bacterial cells can be recovered from the culture medium, the recovered bacterial cells are disrupted by pressure treatment or ultrasonic treatment, and the phosphodiesterase can then be separated and/or purified. As a method for separating and/or purifying phosphodiesterase, a protein separation and/or purification method known per se may be used. Examples of such method include, but are not limited to, centrifugation method, UF concentration method, salting out method, and various chromatography methods using ion exchange resin and the like.

When the phosphodiesterase of the present invention is prepared using a microorganism, the phosphodiesterase of the present invention may or may not be isolated and/or purified from the microorganism. When the phosphodiesterase of the present invention is purified, the purity of the purified phosphodiesterase is not particularly limited.

In one embodiment of the present invention, the phosphodiesterase of the present invention may be a nuclease or a ribonuclease.

### 2. Enzyme agent for degrading composition with high nucleic acid content

The present invention also provides an enzyme agent for degrading compositions with high nucleic acid content, which contains the phosphodiesterase of the present invention (hereinafter sometimes referred to as the "enzyme agent for degradation of the present invention").

As described above, the phosphodiesterase of the present invention is characterized by higher GMP production capacity, under high substrate concentrations, than existing phosphodiesterases. Therefore, by utilizing this aspect of the phosphodiesterase of the present invention, nucleic acids can be degraded more efficiently, in compositions containing high concentrations of nucleic acids, than existing phosphodiesterases.

The amount of the phosphodiesterase of the present invention in the enzyme agent for degradation of the present invention is not particularly limited. The amount of phosphodiesterase is generally 0.0001 to 100% by weight, preferably 0.001 to 100% by weight, with respect to the weight of the enzyme agent of the present invention.

The enzyme agent for degradation of the present invention may contain components other than the phosphodiesterase of the present invention. Such components include, but are not limited to, additives such as stabilizers, buffers, preservatives, excipients, suspending agents, antiseptics, pH adjusters, physiological saline, and the like. Excipients include, but are not limited to, starch, dextrin, maltose, trehalose, lactose, D-glucose, sorbitol, D-mannitol, sucrose, glycerin, and the like. Buffers include, but are not limited to, phosphate, citrate, acetate, and the like. Stabilizers include, but are not limited to, propylene glycol, ascorbic acid, and the like. Preservatives include, but are not limited to, phenol, benzalkonium chloride, benzyl alcohol, chlorobutanol, methylparaben, and the like. Antiseptics include, but are not limited to, ethanol, benzalkonium chloride, parahydroxybenzoic acid, chlorobutanol, and the like. pH adjusters include, but are not limited to, acetate buffer, phosphate buffer, MES buffer, HEPES buffer, PIPES buffer, Bis-Tris buffer, MOPS buffer, and the like. Only one kind of these additives may be contained alone or plural kinds thereof may be contained in combination.

The form of the enzyme agent for degradation of the present invention is not particularly limited as long as the biological activity of the phosphodiesterase of the present invention is maintained, and may be any of solid, semi-solid, powder, slurry, and liquid.

The composition with high nucleic acid content to which the enzyme agent for degradation of the present invention is applied refers to a composition containing a large amount of nucleic acid to the extent that it reduces the GMP production capacity of existing phosphodiesterases (e.g., phosphodiesterase derived from the genus Penicillium) through substrate inhibition. The composition with high nucleic acid content may be a composition that inherently contains many nucleic acids (e.g., high nucleic acid yeast), or may be a composition in which nucleic acid has been added externally to increase the nucleic acid concentration.

The concentration of nucleic acid contained in the composition with high nucleic acid content is not particularly limited as long as it is sufficient to reduce the activity of existing phosphodiesterases. It is generally 35 mg/mL or more, preferably 40 mg/mL or more, 45 mg/mL or more, 50 mg/mL or more, 55 mg/mL or more, 60 mg/mL or more, 65 mg/mL or more, 70 mg/mL or more, 75 mg/mL or more, 80 mg/mL or more, 85 mg/mL or more, 90 mg/mL or more, 95 mg/mL or more, 100 mg/mL or more, 110 mg/mL or more, 120 mg/mL or more, 130 mg/mL or more, 140 mg/mL or more, 150 mg/mL or more, 160 mg/mL or more, 170 mg/mL or more, 180 mg/mL or more, 190 mg/mL or more, 200 mg/mL or more, 210 mg/mL or more, 220 mg/mL or more, 230 mg/mL or more, 240 mg/mL or more, 250 mg/mL or more, 260 mg/mL or more, 270 mg/mL or more, 280 mg/mL or more, 290 mg/mL or more, 300 mg/mL or more, 350 mg/mL or more, 400 mg/mL or more, 500 mg/mL or more, 600 mg/mL or more, 700 mg/mL or more, 800 mg/mL or more, 900 mg/mL or more, 1000 mg/mL or more, 1100 mg/mL or more, or 1200 mg/mL or more, but is not limited to these. The upper limit value of the nucleic acid concentration is not particularly limited and is generally 10000 mg/mL or less, preferably 9000 mg/mL or less, 8000 mg/mL or less, 7000 mg/mL or less, 6000 mg/mL or less, 5000 mg/mL or less, 4000 mg/mL or less, 3500 mg/mL or less, 3400 mg/mL or less, 3300 mg/mL or less, 3200 mg/mL or less, 3100 mg/mL or less, 3000 mg/mL or less, 2900 mg/mL or less, 2800 mg/mL or less, 2700 mg/mL or less, 2600 mg/mL or less, 2500 mg/mL or less, 2400 mg/mL or less, 2300 mg/mL or less, 2200 mg/mL or less, 2100 mg/mL or less, 2000 mg/mL or less, 1900 mg/mL or less, 1800 mg/mL or less, 1700 mg/mL or less, 1600 mg/mL or less, 1500 mg/mL or less, 1400 mg/mL or less, 1300 mg/mL or less, 1200 mg/mL or less, 1100 mg/mL or less, 1000 mg/mL or less, 900 mg/mL or less, 800 mg/mL or less, 700 mg/mL or less, 600 mg/mL or less, 500 mg/mL or less, 400 mg/mL or less, 300 mg/mL or less, 250 mg/mL or less, 200 mg/mL or less, 190 mg/mL or less, 180 mg/mL or less, 170 mg/mL or less, 160 mg/mL or less, 150 mg/mL or less, 140 mg/mL or less, 130 mg/mL or less, 120 mg/mL or less, 110 mg/mL or less, 100 mg/mL or less, 90 mg/mL or less, 80 mg/mL or less, 70 mg/mL or less, 60 mg/mL or less, 50 mg/mL or less, 40 mg/mL or less, 30 mg/mL or less, 20 mg/mL or less, 10 mg/mL or less, 9 mg/mL or less, 8 mg/mL or less, 7 mg/mL or less, 6 mg/mL or less, 5 mg/mL or less, 4 mg/mL or less, 3 mg/mL or less, 2 mg/mL or less, or 1 mg/mL or less, but is not limited to these.

The amount of the enzyme agent for degradation of the present invention to be added to a composition with a high nucleic acid content may vary depending on the amount of the phosphodiesterase of the present invention incorporated into the enzyme agent for degradation of the present invention, the enzyme reaction conditions, and the like, and may be appropriately determined using a method known per se.

### 3. Enzyme agent for producing composition containing GMP

The present invention also provides an enzyme agent for producing a composition containing GMP, containing the phosphodiesterase of the present invention (hereinafter sometimes referred to as the "enzyme agent for producing a GMP-containing composition of the present invention").

The phosphodiesterase of the present invention can efficiently produce GMP by using nucleic acid as substrate, particularly under the conditions of high substrate concentration. By utilizing this property, the phosphodiesterase of the present invention can be preferably used in the production of GMP-containing compositions, including yeast extract. As described above, moreover, since the phosphodiesterase of the present invention is less prone to substrate inhibition even under high substrate concentration conditions, compositions with a high GMP content can be produced.

The amount of the phosphodiesterase of the present invention, the components other than the phosphodiesterase, the form thereof, and the like in the enzyme agent for producing a GMP-containing composition of the present invention, are the same as those described for the enzyme agent for degradation of the present invention.

The amount of the enzyme agent for producing a GMP-containing composition of the present invention to be added may vary depending on the amount of the phosphodiesterase of the present invention incorporated into the enzyme agent for producing a GMP-containing composition of the present invention, the reaction conditions, and the like, and may be appropriately determined using a method known per se.

The subject to which the enzyme agent for producing a GMP-containing composition of the present invention is applied is also not particularly limited. However, since the phosphodiesterase of the present invention contained in the enzyme agent for producing a GMP-containing composition of the present invention aims to produce GMP by using nucleic acid as a substrate, it is essential that the subject to which the enzyme agent for producing a GMP-containing composition of the present invention is applied contains nucleic acid. The nucleic acid may be derived from the subject or may be added externally. One example of such subject is yeast extract. A process for producing yeast extract by using the enzyme agent for producing a GMP-containing composition of the present invention is described below as one embodiment of the enzyme agent of the present invention.

Yeast grown using a conventional method is recovered, the recovered yeast is disrupted using a homogenizer or ultrasonic treatment, and intracellular components are extracted together with a solvent. The obtained extract is filtered to remove solid contents, and then the obtained liquid is concentrated by subjecting to evaporation concentration or centrifugation to prepare a yeast extract. The phosphodiesterase of the present invention is added to the prepared yeast extract and reacted for 1 to 24 hr under the conditions of 50-90°C and pH 4.0-7.0, whereby nucleic acids (particularly RNA) contained in the yeast extract can be converted to GMP. The timing of the action of the phosphodiesterase of the present invention on the yeast or yeast extract is not particularly limited as long as the desired effect is achieved. It may be during any of a step of disrupting yeast, a step of extracting the intracellular component together with a solvent, a step of filtering the extract to remove solid contents, or a concentration step. In one embodiment of the present invention, the yeast used as a raw material may be yeast characterized by containing a large amount of nucleic acids. This aspect can be interpreted as a method for producing a yeast extract containing a high concentration of GMP, which includes a step of allowing the phosphodiesterase of the present invention to act on yeast or yeast extract (hereinafter sometimes referred to as the "production method of yeast extract of the present invention"). The GMP content of such yeast extract is generally 1 mg/mL or more, preferably 2 mg/mL or more, 3 mg/mL or more, 4 mg/mL or more, 5 mg/mL or more, 6 mg/mL or more, 7 mg/mL or more, 8 mg/mL or more, or 9 mg/mL or more, but is not limited to these. The upper limit is also not particularly limited and is generally, 1000 mg/mL or less, preferably 900 mg/mL or less, 800 mg/mL or less, 700 mg/mL or less, 600 mg/mL or less, 500 mg/mL or less, 450 mg/mL or less, 400 mg/mL or less or 350 mg/mL or less, 300 mg/mL or less, 250 mg/mL or less, 200 mg/mL or less, 190 mg/mL or less, 180 mg/mL or less, 170 mg/mL or less, 160 mg/mL or less, 150 mg/mL or less, 140 mg/mL or less, 130 mg/mL or less, 120 mg/mL or less, 110 mg/mL or less, 100 mg/mL or less, 90 mg/mL or less, 80 mg/mL or less, 70 mg/mL or less, 60 mg/mL or less, 50 mg/mL or less, 40 mg/mL or less, 30 mg/mL or less, 20 mg/mL or less, 10 mg/mL or less, 9 mg/mL or less, 8 mg/mL or less, 7 mg/mL or less, 6 mg/mL or less, 5 mg/mL or less, 4 mg/mL or less, 3 mg/mL or less, 2 mg/mL or less, or 1 mg/mL or less, but is not limited to these.

A specific one embodiment of the production method of yeast extract of the present invention is as follows:
a method for producing yeast extract including the following steps:
a first step of preparing a composition containing nucleic acid;
a second step of reacting the nucleic acid-containing composition prepared in the first step with the phosphodiesterase of the present invention;
a step of recovering the GMP-containing composition prepared in the second step.

The nucleic acid used in this embodiment may be chemically synthesized or derived from a living organism. The biological species from which the nucleic acid is derived is not particularly limited, and may be preferably yeast.

In another embodiment, the concentration of nucleic acid in the nucleic acid-containing composition prepared in the first step is preferably high. Such nucleic acid concentration is not particularly limited as long as it is sufficient to reduce the activity of existing phosphodiesterases. It is generally 35 mg/mL or more, preferably 40 mg/mL or more, 45 mg/mL or more, 50 mg/mL or more, 55 mg/mL or more, 60 mg/mL or more, 65 mg/mL or more, 70 mg/mL or more, 75 mg/mL or more, 80 mg/mL or more, 85 mg/mL or more, 90 mg/mL or more, 95 mg/mL or more, 100 mg/mL or more, 110 mg/mL or more, 120 mg/mL or more, 130 mg/mL or more, 140 mg/mL or more, 150 mg/mL or more, 160 mg/mL or more, 170 mg/mL or more, 180 mg/mL or more, 190 mg/mL or more, 200 mg/mL or more, 210 mg/mL or more, 220 mg/mL or more, 230 mg/mL or more, 240 mg/mL or more, 250 mg/mL or more, 260 mg/mL or more, 270 mg/mL or more, 280 mg/mL or more, 290 mg/mL or more, 300 mg/mL or more, 350 mg/mL or more, 400 mg/mL or more, 500 mg/mL or more, 600 mg/mL or more, 700 mg/mL or more, 800 mg/mL or more, 900 mg/mL or more, 1000 mg/mL or more, 1100 mg/mL or more, or, 1200 mg/mL or more, but is not limited to these. The upper limit value of the nucleic acid concentration is not particularly limited, and is generally 10000 mg/mL or less, preferably 9000 mg/mL or less, 8000 mg/mL or less, 7000 mg/mL or less, 6000 mg/mL or less, 5000 mg/mL or less, 4000 mg/mL or less, 3500 mg/mL or less, 3400 mg/mL or less, 3300 mg/mL or less, 3200 mg/mL or less, 3100 mg/mL or less, 3000 mg/mL or less, 2900 mg/mL or less, 2800 mg/mL or less, 2700 mg/mL or less, 2600 mg/mL or less, 2500 mg/mL or less, 2400 mg/mL or less, 2300 mg/mL or less, 2200 mg/mL or less, 2100 mg/mL or less, 2000 mg/mL or less, 1900 mg/mL or less, 1800 mg/mL or less, 1700 mg/mL or less, 1600 mg/mL or less, 1500 mg/mL or less, 1400 mg/mL or less, 1300 mg/mL or less, 1200 mg/mL or less, 1100 mg/mL or less, 1000 mg/mL or less, 900 mg/mL or less, 800 mg/mL or less, 700 mg/mL or less, 600 mg/mL or less, 500 mg/mL or less, 400 mg/mL or less, 300 mg/mL or less, 250 mg/mL or less, 200 mg/mL or less, 190 mg/mL or less, 180 mg/mL or less, 170 mg/mL or less, 160 mg/mL or less, 150 mg/mL or less, 140 mg/mL or less, 130 mg/mL or less, 120 mg/mL or less, 110 mg/mL or less, 100 mg/mL or less, 90 mg/mL or less, 80 mg/mL or less, 70 mg/mL or less, 60 mg/mL or less, 50 mg/mL or less, 40 mg/mL or less, 30 mg/mL or less, 20 mg/mL or less, 10 mg/mL or less, 9 mg/mL or less, 8 mg/mL or less, 7 mg/mL or less, 6 mg/mL or less, 5 mg/mL or less, 4 mg/mL or less, 3 mg/mL or less, 2 mg/mL or less, or 1 mg/mL or less, but is not limited to these.

According to the production method of yeast extract of the present invention, liquid or solid (typically, powdered, granular, etc.) yeast extract can be obtained. The yeast extract obtained by the production method of yeast extract of the present invention can be used to enhance or adjust the taste of various foods and beverages. Examples of applicable foods and beverages include processed seafood products (chikuwa, kamaboko, hanpen, dried squid, dried fish, salted fish, fish sausage, tsukudani, canned goods, etc.), processed meat products (ham, bacon, sausage, jerky, corned beef, formed meat, etc.), processed vegetables (canned and bottled vegetables, processed tomatoes, processed mushrooms, pickled vegetables, dried vegetables, tsukudani vegetables, etc.), noodles and breads (various types of noodles, bread, sweet rolls, etc.), processed grain products (cereal, oatmeal, muesli, processed rice products, wheat gluten, barley tea, etc.), dairy products (milk, processed milk, dairy drinks, concentrated milk, milk powder, condensed milk, fermented milk, lactic acid bacteria drinks, butter, cheese, ice cream, etc.), processed fruit products (canned and bottled fruit, jam, marmalade, dried fruit, etc.), sweets and desserts (biscuits, baked goods, rice crackers, fried sweets, fresh Japanese sweets, fresh Western sweets, semi-fresh sweets, dried Japanese sweets, candy, chocolate, chewing gum, snacks, frozen desserts, etc.), beverages (soft drinks, carbonated drinks, fruit juice drinks, coffee drinks, vegetable juice drinks, tea drinks, non-alcoholic drinks, alcoholic drinks, etc.), seasonings (tare sauces, soup bases, dressings, sauces, etc.), soups, roux (curry roux, stew roux, etc.), nutritional supplements and beverages (protein powder, protein drinks, supplements, energy drinks, etc.), pet food, nutritional supplements for pets.

### 4. Nucleic acid, vector, and cell

The present invention also provides the nucleic acid represented by SEQ ID NO:3 or 4, or a nucleic acid having 60% or more sequence identity with the nucleic acid (hereinafter sometimes referred to as the "nucleic acid of the present invention"); a vector containing the nucleic acid (hereinafter sometimes referred to as the "vector of the present invention"); or a cell containing the vector (hereinafter sometimes referred to as the "cell of the present invention").

The nucleic acid of the present invention encodes the aforementioned phosphodiesterase of the present invention. The amino acid sequence of the phosphodiesterase of the present invention is as described above. When the amino acid sequence is known, one skilled in the art can determine the nucleic acid sequence encoding the amino acid sequence by using a method known per se. SEQ ID NO:3 encodes the phosphodiesterase of the present invention (without a signal sequence), and SEQ ID NO:4 encodes the phosphodiesterase of the present invention (with a signal sequence).

In the nucleic acid of the present invention, codons may be replaced with degenerate codons as long as it encodes the phosphodiesterase of the present invention. In other words, the nucleic acid of the present invention may not be SEQ ID NO:3 or SEQ ID NO:4 itself, as long as it encodes the phosphodiesterase of the present invention.

In another embodiment, the nucleic acid of the present invention contains or consists of the nucleic acid represented by SEQ ID NO:3 or 4, or a nucleic acid having 60% or more, preferably 70% or more, 80% or more, or 90% or more (more preferably 91% or more, 92% or more, 93% or more, 94% or more, 95% or more, 96% or more, 97% or more, 98% or more, or 99% or more) identity with the nucleic acid. The nucleic acid containing or consisting of a nucleic acid having 60% or more, preferably 70% or more, 80% or more, or 90% or more (more preferably 91% or more, 92% or more, 93% or more, 94% or more, 95% or more, 96% or more, 97% or more, 98% or more, or 99% or more) identity with the nucleic acid represented by SEQ ID NO:3 or 4 may be a nucleic acid encoding a phosphodiesterase having GMP production capacity equal to or greater than that of a phosphodiesterase consisting of the amino acid sequence represented by SEQ ID NO:1 or 2. The identity (%) of nucleic acid sequences can be determined by a method known per se.

In one embodiment, the nucleic acid of the present invention may be one in which the nucleic acid encoding the amino acid at the active center of the phosphodiesterase of the present invention (i.e., GACTCGTATCGGGCCACGGCA, SEQ ID NO: 6 (corresponding to the 133-153 positions in SEQ ID NO: 3 or the 193-213 positions in SEQ ID NO: 4)) is not altered and is altered at other positions.

In another embodiment, the nucleic acid of the present invention may be one in which the nucleic acid encoding the amino acid at the active center is altered from SEQ ID NO:6 as long as the amino acid sequence at the active center of the phosphodiesterase of the present invention (i.e., DSYRATA (SEQ ID NO:5)) is maintained.

The vector of the present invention may be any as long as it contains the nucleic acid of the present invention. The vector may be a cloning vector or an expression vector. Cloning vector and expression vector may be selected appropriately depending on the host cell. In one embodiment, for example, when E. coli is the host cell, cloning vectors include, but are not limited to, M13 vectors, pUC vectors, pBR322, pBluescript, pCR-Script, pGEM-T, pDIRECT, pT7, and the like. Expression vectors include, but are not limited to, pGEX, pET, and the like.

The cell of the present invention is not particularly limited as long as it is capable of amplifying the vector of the present invention and expressing the phosphodiesterase of the present invention when the vector of the present invention is introduced. Examples of such cell include, but are not limited to, E. coli, yeast, animal cells, plant cells, insect cells, and the like.

The present invention is explained more specifically in the following by referring to Examples, which are merely examples and the present invention is not limited thereto.

### [Example]

### [Preparation of phosphodiesterase]

Leptographium procerum NBRC110178 strain (obtained from the National Institute of Technology and Evaluation (NITE) Biotechnology Center (NBRC)) was cultured in PDA medium (manufactured by Becton Dickinson and Company) and then water was added to obtain a soak solution. The soak solution was filtered through filter paper and then ultrafiltered to remove insoluble components and impurities, and phosphodiesterase was concentrated to give an enzyme solution.

As Comparative Example, commercially available phosphodiesterase derived from the genus Penicillium (manufactured by Amano Enzyme Inc.) was used.

The activity of each phosphodiesterase was measured based on the phosphodiesterase activity test method in the 9th edition of Japan's Specifications and Standards for Food Additives.

A substrate solution was prepared by dissolving 20 mg of adenosine-3'-monophosphate in 10 mL of sodium barbital hydrochloride buffer (pH 5.0) and filtering through a membrane filter (0.45 µm). Amidol reagent was prepared by weighing 0.50 g of 2,4-diaminophenol dihydrochloride and 10.0 g of sodium bisulfite, dissolving by adding water to make 50 mL, and filtering. 0.4 mL of the substrate solution prepared when in use was left at 70±0.5°C for exactly 5 min, 0.1 mL of the enzyme solution was added and the mixture was left at 70±0.5°C for exactly 15 min. 4 mL of 6% perchloric acid reagent, 0.4 mL of Amidol reagent, and 0.2 mL of hexaammonium heptamolybdate tetrahydrate solution (8.3 → 100) were mixed. The mixture was left in running water for 15 min, and the absorbance at 750 nm was measured using water as a control.

### [Experimental Example 1] Measurement of enzyme activity

Solutions were prepared by adding ribonucleic acid (yeast-derived; manufactured by FUJIFILM Wako Pure Chemical Corporation) and the phosphodiesterase obtained in Example 1 or the phosphodiesterase of Comparative Example so as to achieve the various substrate concentrations and enzyme activities per substrate shown in Table 1, and they were reacted at 70°C for 180 min. After completion of the reaction, the enzyme was inactivated by boiling at 100°C for 5 min. Each reaction solution was filtered through a 0.45 µm hydrophilic mixed cellulose ester (MCE) membrane filter, and then GMP was fractionated and quantified using Shim-Pack WAX-1 (manufactured by SHIMAZU: 4.0 mm ID, x 50 mm L 3 µm, P/N: 228-16225-91). Furthermore, the amount of GMP produced by the enzyme of the present invention (phosphodiesterase derived from the genus Leptographium) was calculated when the amount of GMP produced by a commercially available product (phosphodiesterase derived from the genus Penicillium) was set to 1 (GMP production ratio). The results are shown in Table 1.

**[Table 1]**

| ribonucleic acid (mg/mL) | activity added (U/mg-RNA) | genus Leptographium | genus Penicillium | GMP production ratio |
|---|---|---|---|---|
| | | amount of GMP produced (mg/mL) | | |
| 240 | 10.1 | 10.9 | 4.2 | 2.6 |
| 180 | 13.5 | 15.8 | 7.4 | 2.1 |
| 120 | 20.3 | 21.4 | 9.8 | 2.2 |
| 60 | 40.5 | 22.1 | 13.7 | 1.6 |
| 40 | 60.8 | 17.8 | 13.8 | 1.3 |
| 30 | 81 | 13.4 | 12.8 | 1 |
| 22.5 | 108 | 10.2 | 9.4 | 1.1 |
| 15 | 162 | 6.9 | 5.5 | 1.3 |
| 7.5 | 324 | 3.5 | 2.7 | 1.3 |
| 3.75 | 648 | 1.7 | 1.2 | 1.4 |

Compared to the nuclease derived from the genus Penicillium, the nuclease derived from the genus Leptographium showed high GMP production even at high substrate concentrations. It was demonstrated that the present invention can produce high amounts of GMP even at high substrate concentrations of 40 mg/ml or higher.

### [Experimental Example 2] Determination of amino acid sequence of phosphodiesterase

The amino acid sequence of the phosphodiesterase derived from Leptographium procerum used in this Example was determined using known methods. Briefly, Leptographium procerum NBRC110178 strain was first cultured and grown, and the cells were then harvested. The genome was then extracted using a conventional method, and the base sequence corresponding to the phosphodiesterase contained in the microorganism was determined using a next-generation sequencer. The amino acid sequence of the phosphodiesterase was determined from the determined base sequence.

The determined sequences are as follows:
SEQ ID NO:1: amino acid sequence of phosphodiesterase used in this Example (without signal sequence)
SEQ ID NO:2: amino acid sequence of phosphodiesterase used in this Example (with signal sequence (intact sequence))
SEQ ID NO:3: base sequence encoding amino acid sequence of phosphodiesterase used in this Example (without signal sequence)
SEQ ID NO:4: base sequence encoding amino acid sequence of phosphodiesterase used in this Example (with signal sequence)
SEQ ID NO:5: amino acid sequence of active center of phosphodiesterase used in this Example
SEQ ID NO:6: base sequence encoding amino acid sequence of active center of phosphodiesterase used in this Example

### [Industrial Applicability]

According to the present invention, GMP can be produced efficiently from a nucleic acid even under high substrate concentration conditions. According to the present invention, moreover, compositions containing GMP, including yeast extract, can be produced under reaction conditions that could not be adopted when using conventional enzymes. Therefore, the present invention is extremely useful, for example, in the food manufacturing field.

This application is based on a patent application No. 2023-144802 filed in Japan (filing date: September 6, 2023) and a patent application No. 2023-194225 filed in Japan (filing date: November 15, 2023), the contents of which are incorporated in full herein.

## Claims

1. A phosphodiesterase derived from the genus Leptographium, comprising the amino acid sequence represented by SEQ ID NO:1, or an amino acid sequence having 60% or more identity with the amino acid sequence, and having high GMP production capacity even in the presence of a high-concentration nucleic acid.

2. The phosphodiesterase according to claim 1, wherein the genus Leptographium is Leptographium procerum.

3. The phosphodiesterase according to claim 1 or 2, wherein the amount of GMP produced is 20 mg/ml or more when an enzyme reaction is performed under the following conditions:
<conditions>
·ribonucleic acid concentration: 40 mg/ml
·reaction temperature: 70°C
·reaction time: 180 min
·pH of the reaction mixture: 5.5
·amount of phosphodiesterase added per 1 mg of substrate RNA :60.8 U/mg.

4. An enzyme agent for degrading a composition with high nucleic acid content, comprising the phosphodiesterase according to claim 1.

5. The enzyme agent according to claim 4, wherein the concentration of the nucleic acid in the composition with high nucleic acid content is 35 mg/mL or more.

6. An enzyme agent for producing a composition comprising 5'-guanosine monophosphate, comprising the phosphodiesterase according to claim 1.

7. The enzyme agent according to claim 6, wherein the composition comprising 5'-guanosine monophosphate is yeast extract.

8. A method for producing a composition comprising 5'-guanosine monophosphate, comprising contacting the phosphodiesterase according to claim 1 or 2 or the enzyme agent according to any one of claims 4 to 7 with a composition comprising a nucleic acid.

9. The production method according to claim 8, wherein the ratio of the amount of the nucleic acid in the composition to the activity of phosphodiesterase is 0.001 U to 1000 U per 1 mg of the nucleic acid.

10. The production method according to claim 9, wherein the concentration of the nucleic acid in the composition is 35 mg/mL or more.

11. The production method according to claim 8, wherein the composition comprising the 5'-guanosine monophosphate is yeast extract.

12. A GMP-containing composition produced by reacting the phosphodiesterase according to claim 1 or 2 or the enzyme agent according to any one of claims 4 to 7 with a nucleic acid.

13. A nucleic acid represented by SEQ ID NO:3 or 4, or a nucleic acid having 60% or more sequence identity with the nucleic acid.

14. A vector comprising the nucleic acid according to claim 13.

15. A cell comprising the vector according to claim 14.
